# EUROPEAN PATENT APPLICATION

(11) **EP 2 484 201 A1**
(43) Date of publication of application: **08.08.2012**
(21) Application number: 09848588.1
(22) Date of filing: 31.08.2009
(51) Int. Cl.: A01H 1/02, A01H 5/10

(54) **METHOD FOR OBTAINING FEMALE PARENT LINES OF ASTERACEAE FROM HYBRIDS**

(71) Applicant: Empresa Brasileira De Pesquisa Agropecuária - EMBRAPA, Cep: 70770-901 Brasília - Df (BR)
(72) Inventor: CARVALHO, Claudio Guilherme Portela de, 86001-970 Londrina - PR (BR); TOLEDO, José Francisco Ferraz de, 86001-970 Londrina - PR (BR)
(74) Representative: Vossius & Partner
(86) International application number: PCT/BR2009/000277
(87) International publication number: WO 2011/022792

(57) **Abstract**

The present invention presents a method for obtaining female inbred lines from Asteracea hybrids, using the species *Helianthus annuus* as a model. The method of the invention is based on the modification of lines with the fertility restorer gene (Rf), obtained from self-pollination of hybrids, in lines presenting normal cytoplasm and not containing the Rf gene. Further, derived male sterile lines were developed. Through the use of this methodology it was possible to obtain female lines from commercial hybrids of sunflower.

## Description

### Field of the invention

The present invention relates to the field of plant improvement for agricultural and ornamental purposes, specifically for the obtaining of female inbred lines from hybrids of sunflower, based on strategies of crossing between inbred lines of interest.

### Background of the invention

The world agricultural production is traditionally based on the development and the improvement of specific genotypes, according to the environmental characteristics of each region, in order to search for varieties with a higher adaptative potential and, above all, higher productivity. Associated to these characteristics, it can be mentioned the search for higher resistance to pathogens, tolerance to abiotic stress, besides the search for the improvement of characteristics directly related to the final product to which the determined culture is destined. A strategy to develop more agriculturally appealing varieties is the development of hybrids. These hybrids are generally more vigorous and productive than the parents, rendering, this way, a lower commercial value for the parent inbred lines.

The production of the hybrid seeds involves controlled pollination between inbred lines, with sufficient transfer of pollen and limited self fertilization. Many methods have been proposed in order to limit the self fertilization of inbred lines, such as the male sterility induced by chemical agents, genetic male sterility, cytoplasmic male sterility and self incompatibility.

The cytoplasmic male sterility associated to pollen's fertility restorer genes has been the main promoters of the development of commercial hybrids of sunflower *(Helianthus annuus* L.) in the world (MILLER, J.F.; FICK, G.N. The genetics of sunflower. In: SCHNEITER, A.A. (Ed.). Sunflower technology and production. Wisconsin: ASA-CSSA-SSSA, 1997. p.441-495.). This procedure is practical and widely used and enhanced by plant breeders for several cultivated species, as can be observed in the exemplified documents US 4,627,192 (sunflower), US 11/854,665 (rucola), US 5,436,386 (Carthamus), WO01/06845 (Solanacea), WO02/098209 (wheat), US 101344,752 (rice) and WO05/029945 (tree species) and in the revision written by Coimbra, J.L.M., Bertoldo, J.G., Do Vale, N.M. Use of male sterility in the breeding of commercial rice hybrids Revista de Ciências Agroveterinárias. Lages, v.7, n.1, p.61-74, 2008.

In the use of this strategy for the production of commercial hybrids of sunflower, the female parental inbred line is a CMS-HA, which presents the characteristic of cytoplasmic male sterility (cytoplasm of the S type), however, no fertility restorer gene (Rf) in the nucleus. The female parent is, therefore, a male sterile inbred line with the genotype S-rfrf. The male parental line is a RHA inbred line, which can carry a cytoplasm of the normal type (N) or of the S type (sterile), but must necessarily carry the Rf gene. Therefore, the RHA inbred line is male sterile, whether with the S-RfRf genotype or with the N-RfRf genotype. The resulting hybrid is male fertile with the S-Rfrf genotype.

Inbred lines containing the fertility restorer gene (RHA inbred lines) can be developed through self pollination of commercial hybrids, following selection of male sterile plants from the segregating populations (MILLER, J.F.; VICK, B.A. Registration of four high linoleic sunflower germplasms. Crop Science, v.41, p.602, 2001; MILLER, J.F.; GULYA, T.J.; SEILER, G.J. Registration of five fertility restorer sunflower germplasms. Crop Science, v.42, p.989-991, 2002.; MILLER, J.F.; GREEN, C.E.; LI, Y.M.; CHANEY, R.L. Registration of three low cadmium (HA 448, HA 449, and RHA 450) confection sunflower genetic stocks. Crop Science, v.46, p.489-490, 2006; MILLER, J.F.; GULYA, T.J.; VICK, B.A. Registration of imidazolinone herbicide-resistant maintainer (HA 442) and fertility restorer (RHA 443) oilseed sunflower germoplasms. Crop Science, v.46, p.483-484, 2006; MILLER, J.F.; GULYA, T.J. Registration of two restorer (RHA 439 and RHA 440) and one maintainer (HA 441) Sclerotinia-tolerant oilseed sunflower germplasms. Crop Science, v.46, p.482, 2006.). These inbred lines must be submitted to tests of progeny in order to guarantee that they present the genotype S-RfRf. On the other hand, lines presenting cytoplasmic male sterility (CMS-HA lines) can be developed through backcrossing, in which the non recurrent parental is any inbred line with the cytoplasm of the S type (male sterile) and the recurrent one is usually a HA inbred line previously improved through several cycles of selection and that carries the genotype N-rfrf. In genetic improvement programs for sunflower, HA lines are maintainer of CMS-HA lines, and are deriving from other HA lines (MILLER, J.F.; VICK, B.A. Registration of four high linoleic sunflower germplasms. Crop Science, v.41, p.602, 2001; MILLER, J.F.; VICK, B.A. Registration of four mid-range oleic acid sunflower genetic stocks. Crop Science, v.42, p.994, 2002.; MILLER, J.F.; SEILER, G.J. Registrations of five oilseed maintainer (HA 429-HA 433) sunflower germplasm lines. Crop Science, v.43, p.2313-2314, 2003; MILLER, J.F.; GULYA, T.J. Registration of two restorer (RHA 439 and RHA 440) and one maintainer (HA 441) Sclerotinia-tolerant oilseed sunflower germplasms. Crop Science, v.46, p.482, 2006.; MILLER, J.F.; GREEN, C.E.; LI, Y.M.; CHANEY, R.L. Registration of three low cadmium (HA 448, HA 449, and RHA 450) confection sunflower genetic stocks. Crop Science, v.46, p.489-490, 2006; MILLER, J.F.; GULYA, T.J.; VICK, B.A. Registration of imidazolinone herbicide-resistant maintainer (HA 442) and fertility restorer (RHA 443) oilseed sunflower germoplasms. Crop Science, v.46, p.483-484, 2006, MILLER, J.F.; GULYA, T.J.; VICK, B.A. Registration of three maintainer (HA 444 to HA 446) and one restorer (RHA 447) high oleic oilseed sunflower germplasms. Crop Science, v.46, p.484-485, 2006). The classical method of crossing is still widely used in order to identify maintainer and restorer of fertility lines in order to use them in the development of hybrids of sunflower, presenting different sources of male sterile cytoplasm, as preformed in the study published by Reddy, C.V.C.M.; Sinha, B., Reddy, A.V.V. and Redy, Y.R. Maintenance of Male Sterility and Fertility Restoration in Different CMS Sources of Sunflower (Helianthus annuus L.). Asian Journal of Plant Sciences 7(8):762-766, 2008.

The possibility of obtaining HA lines and, consequently, of CMS-HA lines from hybrids, especially commercial ones, as is currently performed for RHA inbreds, is an interesting alternative, since it would lead to a better exploitation of the genetic potential of adapted genotypes with desired traits. In this context, the objective of the present invention is to present a method for production of female inbred lines from hybrids of plants of the Asteraceae family, using sunflower as a model, and the embodiments used herein to obtain the described results, through said method.

### Summary of the invention

The present invention presents a method for obtaining female inbred lines from hybrids of the botanic family Asteraceae, using the species *Helianthus annuus* as a model. In order to obtain these female inbred lines, RHA inbred lines, obtained from the self fertilization of hybrids (commercial or non commercial), must be altered into HA inbred lines and, this way, modify these HA lines into male sterile plants. The said method consists in several steps of crossing and evaluation of the progeny obtained from the proposed crosses, as follows:
a) Crossing any HA inbred from Asteracea *(non recurrent parent),*
   previously emasculated (or subjected to previous pollen sterilization through the use of chemical agents or other mechanisms), with a RHA inbred (recurrent parent), carrying the fertility restorer gene;
b) Generation of a F1, containing 50% of the genotype of the recurrent parent of the step **a;**
c) Backcrossing the F1, previously emasculated (or subjected to previous pollen sterilization through the use of chemical agents or
   other mechanisms), with the recurrent RHA line cited in **a;**
d) Obtaining of backcross 1 (BC1), containing 75% of the phenotype of recurrent parent cited in **a;**
e) Identification of the heterozygotes (Rfrf) and homozygotes (RfRf) of the BC1 generation, obtained on the step **d;**
f) Backcrossing BC1 heterozygote (Rfrf) and homozygote (RfRf) plants, previously emasculated (or subjected to previous pollen sterilization through the use of chemical agents or other mechanisms), with the recurrent parent cited in **a,** in order to obtain the BC2. Only the seed of BC1 heterozygote plants proceed to the next generation;
g) Obtaining of BCₙ generation from the BCₙ₋₁ generation, according to the steps **e** and **f.**
h) After successive backcrossings, identification and self pollination of the heterozygotes (Rfrf) and homozygotes (RfRf) of the BCₙ generation, obtained from the step **g.** The self pollination of Rfrf plants of the BCₙ generation will lead to the obtaining of HA plants (N rfrf) and RHA plants (N RfRf and N Rfrf);
i) Identification and self pollination of HA plants (N rfrf) and RHA plants (N (RfRf and N Rfrf) obtained from the self pollination of Rfrf plants on the step h.
j) Altering a HA plant (N rfrf), obtained on the step **h,** into male sterile plant, after successive backcrossings, wherein the non recurrent parent is any plant presenting cytoplasmic male sterility and the recurrent parent is the HA line (N rfrf). The F1 generation is obtained in the identification step of the HA plants described on the step **i.**

The identification step of the BC1 generation in the claimed method is incorporated to the invention according to the following procedure: fertilization of male sterile plants with pollen produced by BC1; identification of a line produced in BC1 as dominant homozygous for the fertility restorer gene (RfRf), if this generates 100% of fertile plants after the cross; or, the identification of a line produced in BC1 as heterozygous for the fertility restorer gene (Rfrf), if this generates fertile and sterile plants. This process is also performed in the identification step of the BCₙ generation (step **h).**

The identification of HA plants HA (N rfrf) and RHA plants (N (RfRf and N Rfrf), obtained in the step i in the claimed method, is incorporated to the invention according to the following procedure: fertilization of male sterile plants with pollen produced from plants under testing; identification of HA plant (N rfrf) if this generates 100% of sterile plants after the cross; or identification of RHA plant (N RfRf and N Rfrf) if this generates 100% of fertile plants or fertile and sterile plants, in any proportion.

The obtaining of the BC2 lines as mentioned in the step f, as an embodiment to the method of the invention, is performed through the pollination of flowers of the BC1 plants, previously emasculated (or subjected to previous pollen sterilization through the use of chemical agents or other mechanisms), with the pollen of the recurrent RHA line cited in the step **a;** the resulting progeny presents normal cytoplasm and carries the dominant homozygous genotype for the fertility restorer gene; or the resulting progeny can be of BC1 lines containing normal cytoplasm and carrying the genotype of restoration fertility in homozygose or heterozygose.

The method described above still presents as embodiment the fact that the fertilization of male sterile plants is performed with pollen extracted from the outer ring of the heads of BC1 plants, region which has this part of the capitulum removed immediately after the pollen extraction.

Other embodiment of the claimed method reveals that the flowers of BC1 to be pollinated with the pollen from the recurrent RHA line are located in the central region of the head.

A modification of the step **h,** as embodiment to the claimed method, is to self fertilize a set of BCₙ plants (RfRf and Rfrf) and identify and self pollinate HA plants HA (N rfrf) and RHA plants (N RfRf and N Rfrf) deriving from this self fertilized set, as described in the step **i.**

An additional embodiment to the presented method is the use of molecular markers, or any other technology, in order to distinguish / identify heterozygous plants and homozygous plants for the fertility restorer gene.

As embodiments of the invention, it can still be mentioned plants, lines and hybrids produced according to the methods of the invention.

### Brief description of the drawing

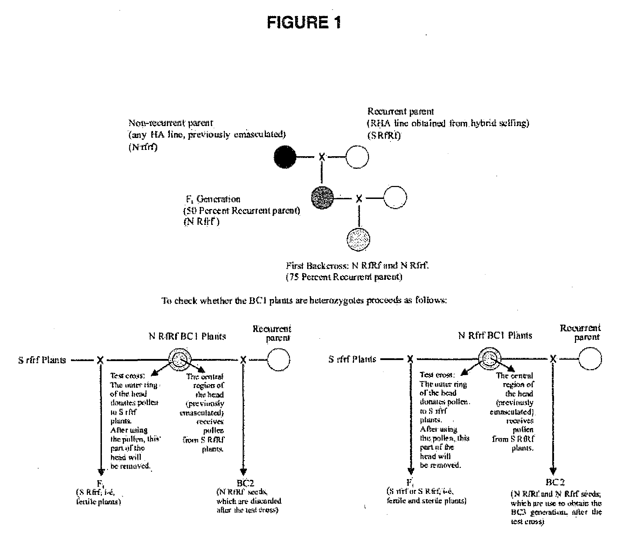

Figure 1. Obtaining of inbred lines of sunflower containing the normal cytoplasm, that are heterozygous or homozygous for the fertility restorer gene Rf.

### Detailed description of the invention

Inbred lines with cytoplasmic male sterility derived from all the RHA lines included in this study were obtained using the methodology proposed in the present invention. The usual procedure for obtaining female lines of sunflower for the production of hybrids demands successive self fertilization of HA lines and incorporation of the cytoplasmic male sterility in these inbred HA plants (MILLER, J.F.; FICK, G.N. The genetics of sunflower. In: SCHNEITER, A.A. (Ed.). Sunflower technology and production. Wisconsin: ASA-CSSA-SSSA, 1997. p.441-495). The methodology described and adopted in this work, besides generating RHA lines and introducing the cytoplasmic male sterility in HA lines, requires the conversion of RHA lines into HA lines, which demands five cycles of reproduction, equivalent to three years (Figure 1). Even though, the possibility of obtaining HA lines and, consequently, CMS-HA lines from hybrids, mainly commercial ones, as is currently performed in order to obtain the RHA lines, is an interesting alternative, since it enables a better exploitation of the genetic potential of the adapted genotypes with desired traits, also for the female parental, with the evident advantages of working in the obtaining of hybrids with improved genotypes for the female as well as for the male. In this context, the objective of the present invention is to present a method of producing female inbred lines from hybrids of plants from the Asteraceae family, using sunflower as a model, and embodiments performed in order to obtain the described results, th rough the cited method.

In the use of the present invention, only the genotypes expressing the restoration of fertility in a monogenic way for the CMS PET1 were used, since in more than 40 sources of identified CMS since the discovered of the PET1 CMS, by Leclercq, in 1968 (Leclercq, P. 1969. Une sterilité cytoplasmique chez le tournesol. Ann. Amelior. Plantes 19:99-106), from a cross between *H.petiolaris* and *H. annuus,* it was found that the restoration of fertility is predominantly controlled by one or two genes (SERIEYS, H. Identification, study and utilization in breeding programs of new CMS sources. Helia, v.19, p.144-160, 1996). Besides this, the great majority of commercial hybrids of sunflower use the source CMS PET1 with a sole fertility restorer gene.

In the recent years, several other sources of cytoplasmic male sterility were described and fertility restorer genes were identified and have been studied (Jan, C.C. and B.A. Vick. Registration of Seven Cytoplasmic Male-Sterile and Four Fertility Restoration Sunflower Germplasms. Crop Sci. 46:1829-1830 (2006).

In an embodiment of the present invention, female inbred lines of sunflower hybrids were developed, from RHA lines with the fertility restorer gene Rf obtained through self fertilization of the hybrids. These RHA lines were initially modified into Ha lines with genotype N-rfrf and, following, into lines with cytoplasmic male sterility with genotype S-rfrf.

In one more embodiment of the invention, the RHA lines were modified into HA lines through successive backcrossing, crossing any HA line (*non recurrent parent*) with an inbred RHA line (recurrent parent) (Figure 1). In the crosses, since the parentals of the female sex (HA and the F1 and BCₙ generations) present a normal cytoplasm, they were previously emasculated. In each generation of backcrossing, flowers emasculated in the central region of the head of sunflower plants of BCₙ, which are homozygous or heterozygous for the Rf gene, received pollen from the recurrent parent in order to produce the seeds of the BCₙ₊₁ generation. Only the seeds of the BCₙ plant heterozygous for the Rf locus proceeded to the next generation. After successive backcrosses, the heterozygotes (Rfrf) and homozygotes (RfRf) of the BCₙ generation were self pollinated. From the self pollination of the Rfrf plants of the BCₙ generation HA plants (N rfrf) and RHA plants (N RfRf and N Rfrf) were obtained. HA plants (N rfrf) and RHA plants (N RfRf and N Rfrf) obtained from the self pollination of Rfrf plants were self pollinated. Following, HA plant (N rfrf) was modified into male sterile plants, through successive backcrossing, in which the non recurrent parent was any plant with cytoplasmic male sterility and the recurrent parent was the HA line (N rfrf) (MILLER, J.F.; FICK, G.N. The genetics of sunflower. In: SCHNEITER, A.A. (Ed.). Sunflower technology and production. Wisconsin: ASA-CSSA-SSSA, 1997. p.441-495). The F1 generation was obtained in the identification step of HA plants. The proceeding followed as any backcrossing system, with male sterile plants crossed to the parental HA.

In order to distinguish the heterozygotes from the homozygous plants for the Rf locus, test crosses were performed to CMS-Ha lines. The offspring of the CMS-HA line crossed to the parental heterozygous for Rf included fertile and sterile plants, while the cross to the parental homozygous for Rf generated only fertile plants. In sunflower, anthesis of flowers in the outer ring of the head happens earlier than that of flowers in the central region. Consequently, for the test crosses, the flowers in the outer ring of the head of BCₙ plants donated pollen to fertilize any male sterile line and it was necessary to be, then, removed from the inflorescence, in order to avoid the fertilization of flowers from the central region, which would be used in order to give continuity to the BCₙ₊₁ backcrosses.

In order to distinguish HA plants (N rfrf) from RHA plants (N RfRf and N Rfrf), test crosses to the CMS-HA lines were performed. The offspring from the CMS-HA line crossed to HA plants (N rfrf) generated only sterile plants, while the cross to the RHA plant (N RfRf and N Rfrf) included fertile and sterile plants.

The viability of transforming the process of extracting female lines from hybrids into a breeding routine is also associated to the number of RHA lines to be modified into HA inbred lines. One way of reducing this number is to evaluate, before modifying, the general and / or specific combining ability of RHA lines by using top crosses to CMS-HA isogenic inbred lines to male of hybrid lines or to genetic male sterility lines or to lines with high capacity of combination. Only the elite lines would be altered, reducing, thus, the required time and labor.

An additional embodiment of the invention is that the test crosses to distinguish heterozygous plants from homozygous plants for the fertility restorer gene can also be facilitated by the use of molecular markers. This is true for the CMS characteristic controlled by a sole gene and is especially useful in those few cases reported in the literature of fertility restoration in sunflower being controlled by a maximum of four genes (VRANCEANU, V.A.; STOENESCU, F.M. Pollen fertility restorer gene from cultivated sunflower (Helianthus annuus L.). Euphytica, v.20, p.536-541, 1971.; ANASHCHENKO, A.V.; DUKA, M.V. Study of the genetic system of CMS-Rf in sunflower (Helianthus annuus L.) III. Restoration of male fertility in the CMS1-based hybrids. Genetika, v.21, p.2005-2010, 1985; IUORAS, M.; VRANCEANU, A.V.; BERVILLE, A. Cytoplasm: nucleus relationships in the CMS pollen fertility restoration in Fundulea 1 (ANT-1) CMS of sunflower. In: INTERNATIONAL SUNFLOWER CONFERENCE, 13., 1992, Pisa. Proceedings.Paris: International Sunflower Association, 1992. p.1072-1077; KURAL, A.; MILLER, J.F. The inheritance of male fertility restoration of the PET2, G1G1 e MAX1 sunflower cytoplasmic male sterility sources. In: INTERNATIONAL SUNFLOWER CONFERENCE, 13., 1992, Pisa. Proceedings. Paris: International Sunflower Association, 1992. p.1107-1112). For the CMS PET1 source, molecular markers (STS) for the Rf₁ (monogenic inheritance) were detected by KUSTERER, B.; PRÜFE, M., LAZARESCU, E. ÖZDEMIR, N.; FRIEDT, W.; HORN, R. Mapping of the restorer gene Rf1 in sunflower (Helianthus annuus L.). Helia, v.25, n.36, p.41-46, jul. 2002.), and then can be used in the identification of Rf₁Rf₁ and Rf₁rf₁ plants).

### Example

CMS-HA lines were derived from 10 inbred RHA lines obtained through self fertilization and selection of plants of several commercial hybrids form Brazil, without recording their origins. Only the genotypes expressing monogenic fertility restoration for the CMS PET1 were used. For the CMS-HA developing process, the RHA lines were initially altered into HA inbreds, through three backcrosses (number sufficient to reach an expected percentage of the recurrent parent above 90%) to the HA 300 line (public USDA-ARS inbred line), used as a non recurrent parent. The RfRf and Rfrf plants obtained from the third backcross generation were, then, selfed and identified through test crosses to the CMS-HA 300 inbred line. This CMS-HA inbred line was also used in order to screen HA (N rfrf) plants from RHA (N RfRf and N Rfrf) plants, from the progeny from Rfrf selfed plants. Following, HA inbred lines were altered into CMS-HA, by the means of three backcrosses to the line CMS-HA 300 used as non recurrent parent, according to the proceedings described in MILLER, J.F.; FICK, G.N.

The genetics of sunflower. In: SCHNEITER, A.A. (Ed.). Sunflower technology and production. Wisconsin: ASA-CSSA-SSSA, 1997. p.441-495. All crosses performed to alter RHA plants in HA plants were performed in a greenhouse environment, in order to obtain a high control of the experimental conditions. However, the crosses for the introduction of the male sterility into HA plants were carried out in the experimental field, as is routinely done in breeding programs.

All the publications and patent applications mentioned in the description are indicative of those skilled in the art to which this invention pertains. All the publications and patent applications are incorporated herein as a matter of reference to the same extent as each individual publication or each patent application was specifically and individually indicated to be incorporated as a matter of reference.

Even though the present invention has been described in some detail by way of illustration and example for the purpose of clarity and understanding, it will be obvious that certain modifications can be practiced within de scope of the attached claims of this description.

## Claims

1. Method of obtaining female inbred lines from Asteracea hybrids, wherein the obtaining of these lines consists in alteration of RHA inbred lines, obtained from the self pollination of hybrids (commercial or non-commercial), into HA inbred lines in order to modify these HA lines into male sterile plants, wherein the said method comprise the following steps:
a) Crossing any HA inbred line from Asteracea *(non recurrent line),* previously emasculated (or subjected to previous pollen sterilization through the use of chemical agents or other mechanisms), with an RHA inbred (recurrent line), carrying the fertility restorer gene;
b) Generating a F1, containing 50% of the genotype of the recurrent line of the step **a;**
c) Backcrossing the F1, previously emasculated (or subjected to previous pollen sterilization through the use of chemical agents or other mechanisms), with the recurrent RHA line cited in **a;**
d) Obtaining the backcross 1 (BC1), containing 75% of the phenotype of recurrent line cited in **a;**
e) Identifying the heterozygotes (Rfrf) and homozygotes (RfRf) of the BC1 generation, obtained on the step **d;**
f) Backcrossing BC1 heterozygote (Rfrf) and homozygote (RfRf) plants, previously emasculated (or subjected to previous pollen sterilization through the use of chemical agents or other mechanisms), with the recurrent parent cited in **a,** in order to obtain the BC2. Only the seed of BC1 heterozygote plants proceed to the next generation;
g) Obtaining BCₙ generation from the BCₙ₋₁ generation, according to the steps **e** and **f.**
h) After successive backcrossings, identifying and selfing the heterozygotes (Rfrf) and homozygotes (RfRf) of the BCₙ generation, obtained from the step **g.** The self pollination of Rfrf plants of the BCₙ generation will lead to the obtaining of HA plants (N rfrf) and RHA plants (N RfRf and N Rfrf);
i) Identifying and selfing HA plants (N rfrf) and RHA plants (N RfRf and N Rfrf) obtained from the self pollination of Rfrf plants on the step **h.**
j) Altering a HA plant (N rfrf), obtained on the step **h,** into a male sterile plant, after successive backcrossings, wherein the non recurrent line is any plant presenting cytoplasmic male sterility and the recurrent line is the HA line (N rfrf). The F1 generation is obtained in the identification step of the HA plants described on the step **i**.

2. Method according to claim 1, wherein the step **e** of claim 1, of identification of the BC1 generation, is performed according to the following:
a) Pollinating male sterile plants with pollen produced from BC1;
b) Identifying a line produced in BC1 as dominant homozygous for the fertility restorer gene (RfRf), if this generates 100% of fertile plants after the crossing cited in the step **a;**
c) Identifying a line produced in BC1 as heterozygote for the fertility restorer gene (Rfrf), if this generates fertile and sterile plants after the crossing cited in the step **a**. This process is also performed in the identification step of BCₙ generation (step h) of claim 1.

3. Method according to claim 1, wherein the step i of claim 1, of identification of HA plants (N rfrf) and RHA plants (N RfRf and N Rfrf), is performed according to the following:
a) Pollinating male sterile plants with pollen produced from HA plants (N rfrf) and RHA plants (N RfRf and N Rfrf);
b) Identifying a HA plant (N rfrf) if this generates 100% of sterile plants after the cross cited in the step **a;**
c) Identifying a RHA plant (N RfRf and N Rfrf) if this generates 100% of fertile plants or fertile and sterile plants, in any proportion superior to zero.

4. Method according to claim 1, wherein the step **f** of claim 1, of the obtaining of BC2 lines, is performed according to the following:
a) Pollinating flowers of BC1 plants, previously emasculated (or subjected to previous pollen sterilization through the use of chemical agents or other mechanisms), with pollen of the recurrent RHA line cited in the step **a** of claim 1;
b) Obtaining a BC2 line containing normal cytoplasm and carrying the dominant homozygote genotype for the fertility restorer gene, from the cross according to **a**, performed with a BC1 line, identified according to the step **b** of claim 2;
c) Obtaining a BC2 line containing a normal cytoplasm and carrying the genotype of fertility restoration in homozygose or heterozygose, from the cross according to **a**, performed with a BC1 line, identified according to the step **c** of claim 2.

5. Method according to claim 1, wherein the pollination of male sterile plants is performed with pollen extracted from the outer ring of the heads of BC1 plants, region which, after having the pollen extracted, has this part of the capitulum immediately removed.

6. Method according to claim 1, wherein the flowers of the BC1 to be pollinated with the pollen of the recurrent RHA line are located in the central region of the head.

7. Method according to claim 1, wherein said method is a modification of the step **h** of the said claim, wherein a set of BCₙ plants (RfRf and Rfrf) is selfed and HA plants (N rfrf) and RHA plants (N RfRf and N Rfrf) from this selfed set are identified and self pollinated, as described in the step i.

8. Method according to claim 1, wherein molecular markers, or any other technology, are used in order to distinguish / identify heterozygous plants and homozygous plants for the fertility restorer gene.

9. Method according to claim 1, wherein the Asteracea lines used are of *Helianthus annuus.*

10. Plant of the Asteracea family obtained from commercial hybrids, wherein said plant is obtained through the method according to claim 1.

11. Method for altering RHA lines, with the fertility restorer gene, into HA lines, wherein these HA lines are obtained from the self pollination of the BCₙ line heterozygous for the Rf gene, obtained according to the step **c** of claim 4.

12. Method according to claim 10, wherein the Asteracea lines used are of *Helianthus annuus.*

13. Female inbred line of the Asteracea family, wherein said inbred is obtained by the means of the method according to claim 11.
